Europäisches Patentamt

(19) European Patent Office

Office européen des brevets

(11) Publication number: **0 055 023**
A2

# (12) EUROPEAN PATENT APPLICATION

(21) Application number: **81305411.1**

(22) Date of filing: **16.11.81**

(51) Int. Cl.³: **A 61 L 15/06, C 09 J 3/00**

(30) Priority: **18.12.80 US 217711**

(43) Date of publication of application: **30.06.82**
**Bulletin 82/26**

(84) Designated Contracting States: **AT BE CH DE FR GB IT LI LU NL SE**

(71) Applicant: **E.R. Squibb & Sons, Inc.,**
**Lawrenceville-Princeton Road, Princeton,**
**N.J. 08540 (US)**

(72) Inventor: **Riffkin, Charles, 12C Basswood Plaza,**
**Cranbary New Jersey (US)**

(74) Representative: **Thomas, Roger Tamlyn et al, D. Young &**
**Co. 10 Staple Inn, London WC1V 7RD (GB)**

(54) **Antiseptic containing adhesive composition.**

(57) An adhesive composition comprising a pressure sensitive rubbery elastomer having one or more water soluble or water swellable hydrocolloids and one or more antiseptic or germicidal agents dispersed therein. This adhesive composition can be employed in various medical bandage type products.

EP 0 055 023 A2

ANTISEPTIC CONTAINING
ADHESIVE COMPOSITION

This invention is directed to an antiseptic or germicide containing adhesive composition that is useful for various medical purposes. The adhesive composition comprises a pressure sensitive rubbery elastomer having intimately dispersed therein one or more water soluble or water swellable hydrocolloids and one or more antiseptic or germicidal agents.

This invention is directed to the discovery that by incorporating one or more antiseptic or germicidal agents within an adhesive composition the potential for bacterial overgrowth is considerably diminished and in many cases entirely eliminated while at the same time not interfering with the healing qualities of the bandage.

The adhesive composition comprises a pressure sensitive rubbery elastomer material having intimately dispersed therein one or more water soluble or water swellable hydrocolloids and one or more antiseptic or germicidal agents. This adhesive composition can have a continuous or discontinuous polymeric film such as polyethylene, polypropylene, polyvinylchloride, polyurethane, etc., laminated to one surface as described in United States Patents 3,339,546 and 4,192,785 or it can be included in a multi-layered bandage having an intermediate polymeric foam layer such as polyurethane foam as described in 3,972,328.

Suitable rubbery elastomers for inclusion within the adhesive composition are natural or synthetic viscous gum-like substances such as natural rubber, silicone rubber, acrylonitrile rubber, polyurethane rubber, polyisobutylenes, etc., with polyisobutylenes being preferred (these are commercially available as Vistanex and Hyvis). The viscous gum-like substance acts as a binder for the hydrocolloid particles and, in addition, renders the final adhesive mass tacky, elastic, and pliable. The elastomeric materials should comprise from about 30% to about 60% by weight of the adhesive composition,

preferably from about 35% to about 50% by weight of the adhesive composition.

Suitable water soluble or water swellable hydrocolloids for inclusion within the adhesive composition are gelatin, pectin, sodium carboxymethylcellulose, guar gum, alginic acid, locust bean gum, karaya, etc., and various mixtures thereof. Preferably the hydrocolloid component is a mixture of pectin, gelatin, and sodium carboxymethylcellulose. The hydrocolloid or mixture of hydrocolloids should comprise from about 30% to about 65% by weight of the adhesive layer, preferably from about 40% to about 60% by weight of the adhesive layer.

Suitable antiseptic and germicidal agents for inclusion within the adhesive composition are iodophors such as povidone iodine, iodine crystals, potassium iodide, etc., and mixtures thereof which are included so as to provide from about 1% to about 5% by weight of free iodine in the adhesive composition; phenolics such as phenol, cresol, chlorthymol, thymol, p-chlorophenol, hexachlorophene, etc., and mixtures thereof which are included at from about 0.5% to about 5% by weight of the adhesive composition; mercurials such as phenylmercuric chloride, phenylmercuric nitrate, phenylmercuric borate, thimerosal, mercury bichloride, nitromersol, etc., and mixtures thereof which are included at from about 0.00001 to about 0.001% by weight of the adhesive composition;

nitrofurazone which is included at from about 0.1% to about 5% by weight of the adhesive composition; and chlorhexidine which is included at from about 0.5% to about 5% by weight of the adhesive composition. The iodophors are the preferred antiseptic agents for inclusion within the adhesive composition.

Various optional ingredients can also be included within the adhesive composition. For example, a cohesive strengthening agent can be included as described in United States Patent 4,192,785, as well as small amounts of a plasticizer such as mineral oil, an antioxidant such as butylated hydroxyanisole, a deodorant or perfume agent.

The adhesive composition is prepared by mixing the hydrocolloids and antiseptic materials in a suitable blender. The viscous substance is then added and mixing is continued until a homogeneous dough-like mass is produced. This mass is extruded or rolled into slab of desired thickness. A thin film of polymeric material can be laminated to one surface and the other side can be covered with silicone coated release paper. The slab can then be cut into wafers of desired size.

Bandages including the antiseptic or germicide containing adhesive composition of this invention are useful in treating various wounds. In particular, such bandages will be beneficial

-5- SA23 0055023

in the treatment of dermal ulcers and in burn therapy.,

Bandages including the antiseptic or germicide containing adhesive composition of this invention can be sterilized by conventional techniques such as gamma irradiation.

The following examples are illustrative of the invention.

### Example 1

An antiseptic containing adhesive composition is prepared containing the following ingredients:

| | | |
|---|---|---|
| Pectin powder | 200 | gms. |
| Gelatin powder | 200 | gms. |
| Sodium carboxymethyl- cellulose powder | 100 | gms. |
| Povidone iodine powder (contains 10% by weight free iodine) | 100 | gms. |
| Polyisobutylene | 400 | gms. |

The gelatin, pectin, sodium, carboxymethylcellulose, and povidone iodine are mixed in a blender until thoroughly dispersed. The polyisobutylene is added and mixing is continued until a dough-like mass is produced. This mass is rolled or extruded into sheets of approximately 1/16 inch thickness. A thin film (about 1 mil.) of polyethylene is laminated to one side and the other is covered with silicone coated release.

## Example 2

An antiseptic containing adhesive composition is prepared containing the following ingredients:

| | | |
|---|---|---|
| Pectin powder | 200 | gms. |
| Gelatin powder | 200 | gms. |
| Sodium carboxymethyl-cellulose powder | 178 | gms. |
| Iodine crystals | 10 | gms. |
| Potassium iodide | 12 | gms. |
| Polyisobutylene | 400 | gms. |

The iodine crystals, potassium iodide, and sodium carboxymethylcellulose are mixed in a blender until thoroughly dispersed. The pectin and gelatin powders are added with continuous stirring. Finally, the polyisobutylene is added with continuous stirring until a dough-like mass is produced. The mass is rolled or extruded into sheets of approximately 1/16 inch thickness. A thin film of polyethylene or other polymeric material is laminated to one side and the other is covered with silicone coated release paper.

## Examples 3 - 11

Following the procedure of Examples 1 and 2, additional antiseptic adhesive compositions can be prepared containing the following ingredients.

| Ingredient (% by weight) | 3 | 4 | 5 | 6 | 7 | 8 | 9 | 10 | 11 |
|---|---|---|---|---|---|---|---|---|---|
| Polyisobutylene | 40 | 40 | 45 | 42. | 40 | 40 | 40 | 45 | 40 |
| Gelatin | – | 20 | 17 | – | 20 | – | 20 | 22 | 20 |
| Pectin | 30 | 20 | 17 | 20 | 20 | 25 | – | 22 | 20 |
| Sodium carboxy-methylcellulose | – | 18 | 16 | – | 19.999 | – | 20 | 10 | 17 |
| Guar gum | 25 | – | – | – | – | 30 | – | – | – |
| Locust bean gum | – | – | – | 35 | – | – | – | – | – |
| Karaya gum | – | – | – | – | – | – | 18 | – | – |
| Iodine (in the form of povidone iodine, crystalline iodine, potassium iodate, etc.) | – | – | – | – | – | 5 | 2 | 1 | 3 |

| Ingredient (% by weight) | 3 | 4 | 5 | 6 | 7 | 8 | 9 | 10 | 11 |
|---|---|---|---|---|---|---|---|---|---|
| Phenol | 5 | – | – | – | – | – | – | – | – |
| Hexachlorophene | – | 2 | – | – | – | – | – | – | – |
| Nitrofurazone | – | – | 5 | – | – | – | – | – | – |
| Chlorhexidine | – | – | – | 3 | – | – | – | – | – |
| Phenylmercuric chloride | – | – | – | – | 0.001 | – | – | – | – |

## Example 12

In this _in vitro_ experiment the antiseptic properties of the bandage prepared according to Example 1 is compared with a control bandage prepared by replacing the povidone iodine in Example 1 with an additional 100 gms. of sodium carboxymethylcellulose.

A microbiological medium is prepared containing:

| | | |
|---|---|---|
| Pancreatic digest of casein | 5.0 | gms. |
| Papaic digest of soybean meal | 1.67 | gms. |
| Sodium chloride | 1.67 | gms. |
| Agar | 15.0 | gms. |
| Distilled water to | 1.0 | liter |

This aqueous mixture is sterilized by autoclave at $121^{O}C$ for 15 minutes and has a pH after sterilization of 7.3.

50 ml. portions of the microbiological medium are placed in Petri dishes (150 x 20 mm) and inoculated separately with the microbs listed below. The medium is inoculated in the ratio of 1.0 ml. of organisms suspension containing at least $10^{8}$ colony forming units per ml. for each liter of medium.

| Staphylococcus aureus | ATCC 6538P |
| Escherichia coli | ATCC 8739 |
| Salmonella dublin | ATCC 15480 |
| Pseudomonas aeruginosa | ATCC 9027 |
| Candida albicans | ATCC 10231 |

Four filter paper discs are placed on the agar surface of each inoculated plate 25 mm. in from the edge at $90^{\circ}$ intervals to each other. 18 mm. circles are cut from both the control and iodinated bandage, two of each for any single seeded Petri dish. The release paper is removed from the samples and two control sample circles and two iodinated sample circles are placed (adhesive side down) on the filter paper discs already on each inoculated Petri dish. A metal weight is placed on top of each sample circle and the Petri dishes are kept at $5^{\circ}$C to prediffuse overnight. After overnight incubation the following results in zones of inhibition are (average of two) obtained.

| | Iodinated product of Example 1 | Control |
|---|---|---|
| S. aureus ATCC 6538P | A 24 mm | No zones |
| | B 27 mm | No zones |
| S. dublin ATCC 15480 | A 21 mm | No zones |
| | B 26 mm | No zones |
| C. albicans ATCC 10231 | A 24 mm | No zones |
| | B 15-24 mm | No zones |
| E. coli ATCC 8739 | 23 mm | No zones |
| P. aeruginosa ATCC 9027 | 24 mm | No zones |

0055023

## CLAIMS

1. An antiseptic adhesive composition comprising from about 30% to about 60% by weight of a natural or synthetic viscous gum-like substance, from about 30% to about 65% by weight of one or more water soluble or water swellable hydrocolloids, and an antiseptically effective amount of one or more substances selected from the group consisting of iodophors, phenolics, mercurials, nitrofurazone, and chlorhexidine.

2. The composition of Claim 1 wherein said viscous gum-like substance is elected from the group consisting of natural rubber, silicone rubber, acrylonitrile rubber, polyurethane rubber, and polyisobutylenes, and said hydrocolloid is selected from the group consisting of gelatin, pectin, sodium carboxymethylcellulose, guar gum, alginic acid, locust bean gum, karaya gum, and mixtures thereof.

3. The composition of Claim 2 wherein said antiseptic substance is one or more iodophors present in an amount such that the adhesive composition contains from about 1% to about 5% by weight of iodine.

4. The composition of Claim 2 wherein said antiseptic substance is one or more phenolics selected from the group consisting of phenol, cresol, thymol, chlorthymol, p-chlorphenol, and hexachlorophene and is present at from about 0.5% to about 5% by weight of said composition,

one or more mercurials selected from the group consisting of phenylmercuric chloride, phenylmercuric nitrate, phenylmercuric borate, thimerosol, mercury bichloride, and nitromersol and is present at from about 0.00001% to about 0.001% by weight of said composition, nitrofurazone present at from about 0.1% to about 5% by weight of said composition, or chlorhexidine present at from about 0.5% to about 5% by weight of said composition.

5.    The composition of Claim 3 wherein said viscous gum-like substance is polyisobutylene and said hydrocolloid is a mixture of pectin, gelatin, and sodium carboxymethylcellulose.

6.    The composition of Claim 5 wherein said polyisobutylene is present at from about 35% to about 50% by weight of said composition and said mixture of hydrocolloids is present at from about 40% to 60% by weight of said composition.

7.    The composition of Claim 6 comprising about 20% by weight of pectin, about 20% by weight of gelatin, about 10% by weight of sodium carboxymethylcellulose, about 10% by weight of povidone iodine, and about 40% by weight of polyisobutylene.

8.    The composition of Claim 6 comprising about 20% by weight of pectin, about 20% by weight of gelatin, about 17.8% by weight of sodium carboxymethylcellulose, about 1% by weight of iodine crystals, about 1.2% by weight of potassium iodide, and about 40% by weight of polyisobutylene.

9. The composition of Claim 4 wherein said viscous gum-like substance is polyisobutylene and said hydrocolloid is a mixture of pectin, gelatin, and sodium carboxymethylcellulose.

10. The composition of Claim 9 wherein said polyisobutylene is present at from about 35% to about 50% by weight of said composition and said mixture of hydrocolloids is present at from about 40% to 60% by weight of said composition.